# EUROPEAN PATENT APPLICATION

(11) **EP 4 560 057 A1**
(43) Date of publication of application: **28.05.2025**
(21) Application number: 23211624.4
(22) Date of filing: 22.11.2023
(51) Int. Cl.: D01F 1/10, D01F 6/84

(54) **POLYCAPROLACTONE-POLYLACTIC ACID ELECTROSPUN MESH FOR DRUG DELIVERY**

(71) Applicant: Vilnius University, 01513 Vilnius (LT); Vilnius University Hospital Santaros Klinikos, 08661 Vilnius (LT)
(72) Inventor: Barasa, Povilas, Vilnius (LT); Simoliunas, Egidijus, Vilnius (LT); Buivydas, Andrius, Vilnius (LT); Grybas, Aivaras, Vilnius (LT); Zilinskaite-Tamasauske, Ramune, Vilnius (LT); Bukelskiene, Virginija, Vilnius (LT); Verkauskas, Gilvydas, Vilnius (LT)
(74) Representative: AAA Law

(57) **Abstract**

The present invention relates to electrospun fiber compositions comprising one or more polymer and one or more therapeutically active molecules, wherein the one or more polymer is a copolymer of poly-lactic acid and poly-caprolactone in a molar ratio of 90:10. The one or more therapeutically active molecules may be selected from anti-inflammatory, anti-fibrotic and antimicrobial agents. Such fiber compositions are advantageous in drug delivery methods by ensuring stable diffusion of the drug molecules, and can also be used in treatment of wounds and/or fibrotic tissue. A method of producing the electrospun fiber compositions is also provided.

## Description

### FIELD OF INVENTION

The invention relates to the drug-loaded electrospun fiber compositions for use in drug delivery to the cells and/or for use in treatment of wounds and/or fibrotic tissue, such as fibrotic tissue of urethral strictures. In particular, electrospun fiber composition made of polycaprolactone-polylactic acid copolymer is used.

### BACKGROUND OF INVENTION

Urethral stricture is a scar or fibrosis of the non-muscular tissue which shortens urethral length and narrows the diameter of the urethra. Often urethral strictures result from urethral trauma, infections, cancer and injuries caused by surgical tools, prostate surgery, urinary catheterization. Common symptoms of urethral stricture may include painful urination, urinary tract infection, slow urine stream, abdominal pain, etc. Women rarely develop urethral strictures while men are more susceptible to them since their urethras are longer and more prone to injury and infection. Strictures involve injury of the urethral epithelium or *corpus spongiosum,* and fibrosis occurs during the subsequent healing process. Stricture tissue is rich in myofibroblasts and multinucleated giant cells which are related to fibrosis and stricture formation (Simsek et al., 2018).

The following treatments of urethral strictures are currently available: urethral dilation, internal urethrotomy and urethral reconstruction. Urethral dilation or internal urethrotomy is usually performed first, when the urethra is widened using a series of gradually larger dilating instruments and a cystoscope (Mundy, 2006). The most common problem of dilation or urethrotomy is the recurrence of the fibrotic stricture formation. If dilation or urethrotomy fails and the fibrosis recurs, urethral reconstruction may be needed. In such cases, open surgery is considered to be the gold standard with success rates of 80-95%, however, minimally invasive therapies are still frequently used (Virasoro et al., 2022). During open surgery, the urethra can be reconstructed by urethroplasty, when after cutting out the scar tissue two ends of the urethra are connected. Unfortunately, it is possible only in case of short urethral strictures for the reason of elasticity limitations and possible erectile dysfunction (Mundy, 2006). The urethra may also be reconstructed using the tongue mucosa (Simonato et al., 2008; A., Srivastava et al., 2013), buccal mucosa grafts or skin flaps from the penis or scrotum (Rashidbenam et al., 2019). Oral mucosa is considered to be the preferred substitution material for the anterior urethra urethroplasty (Ma et al., 2021). However, these methods are very painful for patients especially for children.

Currently a new method of urethral stricture treatment is proposed for patients who have failed prior endoscopic management, namely Optilume^{®} being a urethral drug-coated balloon that dilates the urethral lumen and delivers paclitaxel (drug) directly to the stricture significantly reducing the incidence of stricture recurrence (Elliott et al., 2021; Virasoro et al., 2022). However, this remedy is not universal being unsuitable for the treatment of all strictures especially when external surgical intervention is required. Another way to treat strictures is administering an effective amount of a botulinum toxin directly to the mammal urethra. Alternatively, the botulinum toxin may be administered in conjunction with stenting or a surgical procedure (e.g., grafting) (EP 2 056 866 B1). However, botulinum toxin has inhibitory action on release of neuropeptides, neuromodulation and anti-inflammatory effects, for which reason its application calls for further clinical and basic research (Kuo, 2022).

A lot of experimentation is being conducted in order to apply tissue engineering and therefore in search of materials efficient for urethroplastic treatment. For that purpose, naturally derived scaffolds made of silk fibroin (Sack et al., 2016), collagen (El-Kassaby et al., 2003) and alginate (Klekiel et al., 2020) are tested and evaluated. Also, synthetic polymers are also used in tissue engineering applications due to their biocompatibility and sufficient strength to support engineered constructs intended to be used in urethral reconstruction (Rashidbenam et al., 2019).

Most of the previously discussed treatments are currently not advanced to the point of fully curing fibrotic diseases, they can only inhibit their progression. Moreover, the medication currently in use causes various side effects to the users, depending on the way of taking them. There is data about the undesirable effects of anti-fibrotic drugs such as, for example, pirfenidone (Ruwanpura et al., 2020), nintedanib (Proesmans et al., 2019), or mitomycin C (Kurt et al.,2017). Accordingly, there remains a need for advanced drug application and delivery methods and compositions, that could be used, for example, in fibrotic tissue treatment.

### SUMMARY OF INVENTION

The present invention provides an advantageous drug delivery composition, its use in treating wounds and/or fibrotic tissue, as well as a method of preparation of said composition. In particular, the invention relates to fiber composition prepared from a polylactic acid (PLA) and polycaprolactone (PCL) copolymer, which is laden with one or more therapeutically active molecules. The hereby disclosed fiber composition localizes the release of medication allowing reduction of side effects and also exercises control over the drug release time resulting in higher treatment efficacy. At the same time, the mesh structure of fiber composition facilitates passage of nutrients and cells to the tissue where it is used.

Accordingly, the present invention provides the following:
In a first aspect, the present invention provides an electrospun fiber composition comprising one or more polymers and one or more therapeutically active molecules, wherein the one or more polymer is a copolymer of poly-lactic acid and poly-caprolactone in a molar ratio of 90: 10.

In some embodiments, the one or more therapeutically active molecules are selected from anti-inflammatory, anti-fibrotic and anti-microbial agents. In some embodiments, the anti-fibrotic agent is selected from nintedanib and pirfenidone. In preferred embodiments, the anti-fibrotic agent is pirfenidone. In some embodiments, the one or more therapeutically active molecule is present at a concentration of 0.3-3% (w/w). In further embodiments, the one or more therapeutically active molecule is present at a concentration of 1% (w/w).

In some embodiments, in the fiber composition of the present invention, the poly-lactic acid is selected from poly-L-lactic acid and poly-D-lactic acid. In preferred embodiments, poly-lactic acid is poly-L-lactic acid. In some embodiments, the fiber composition of the present invention is in the form of a fiber delivery platform, a fiber dressing, or a fiber scaffold.

In a second aspect, a pharmaceutical composition comprising the fiber composition of the present invention and a pharmaceutically acceptable carrier is provided.

In a third aspect, the fiber composition of the present invention for use in a method of treatment of wounds and/or fibrotic tissue is provided. In some embodiments, the method is for treatment of fibrotic tissue of urethral strictures or hypospadias.

In a fourth aspect, use of the fiber composition of the present invention for delivery of one or more therapeutically active molecules to the cells is provided.

In a fifth aspect, a method for the preparation of the fiber composition of the present invention is provided, the method, comprising: 1) preparing a solution of a copolymer poly-lactic acid and poly-caprolactone and one or more therapeutically active molecules, and 2) subjecting the solution to an electrospinning step, wherein the solution of step 1) is prepared in dichlormethane. In some embodiments, in the method for the preparation of the fiber composition of the present invention the copolymer is present at a concentration of 5-20% (w/v), and the one or more therapeutically active molecule is present at a concentration of 0.03-0.3% (w/v).

### BRIEF DESCRIPTION OF FIGURES

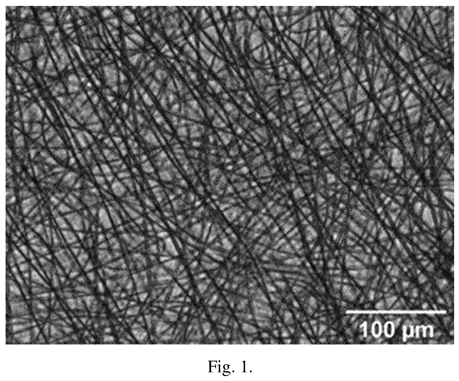

Fig. 1 shows a light microscopy photograph of PFD-loaded electrospun PCL-PLA mesh.

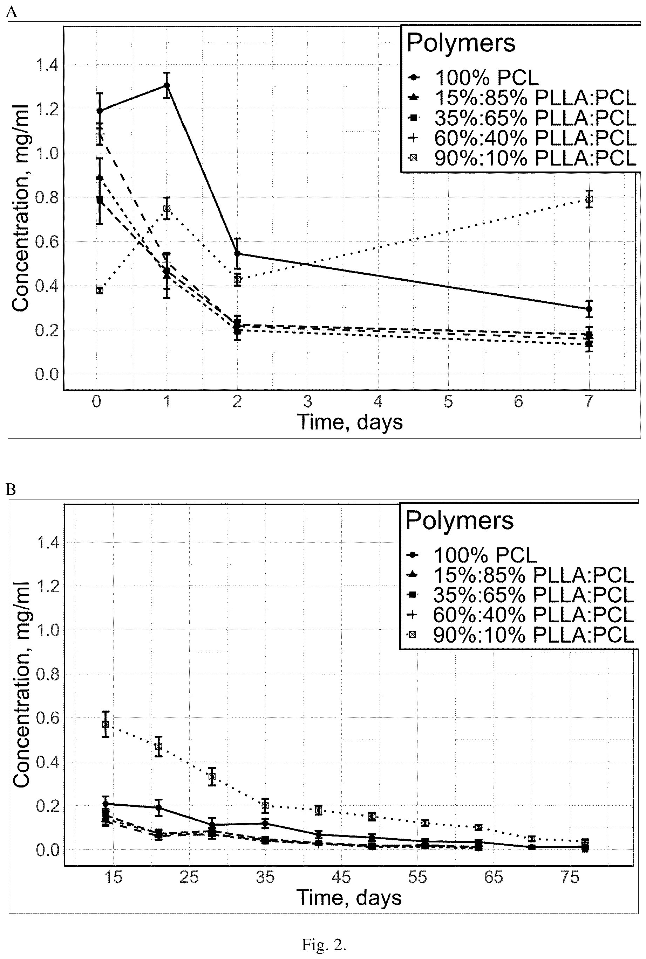

Fig. 2 shows PFD release profiles of different carrier polymers: A - released PFD concentrations during first week; B - released PFD concentrations up to week 11.

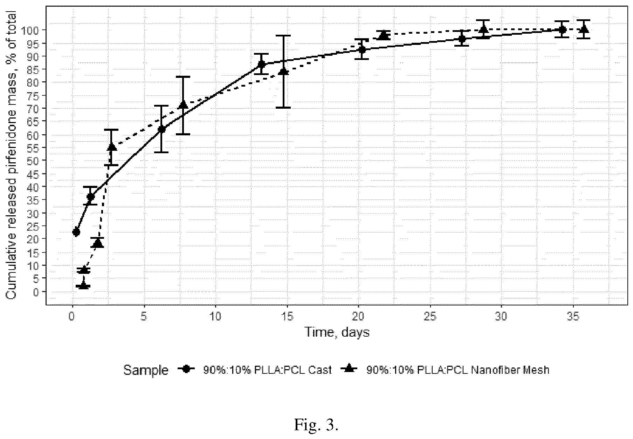

Fig. 3 shows the cumulative release of pirfenidone from samples of cast PCL-PLA and the PCL-PLA mesh. The cumulative release was calculated as a percentage of pirfenidone mass released into the medium at the day of measurement relative to the final calculated mass of released pirfenidone. Squares and triangles represent the released mass percentage means of cast and electrospun samples (error bars denote the SD values), respectively, with solid and dashed lines connecting the measurements.

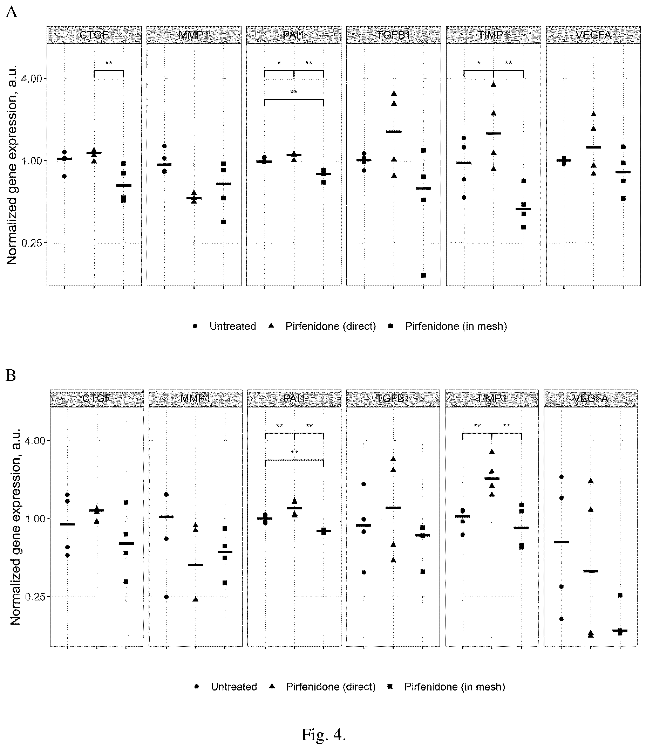

Fig. 4 is a comparison of the effect of directly applied PFD and PFD-laden mesh on fibrosis-related gene expression in primary human myofibroblasts (HMF): A - gene expression levels measured 1 day after application, B - gene expression levels measured 1 week after application. Calculations were normalized to the related gene expression levels measured in untreated cells; bars represent medians of the measurements, and different shapes correspond to different samples. Star (*) denotes statistically significant (p <0.05) differences, and two stars (**) denotes statistically significant (p <0.01) differences between the groups, evaluated via Conover-Iman test.

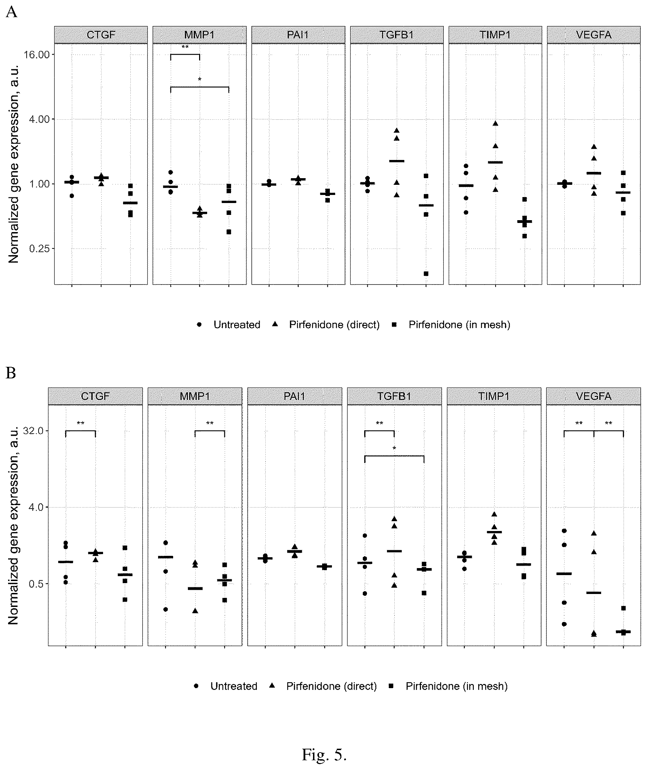

Fig. 5. is a comparison of the effect of directly applied PFD and PFD-laden mesh on gene expression in human prostate cancer cell line WPMY-1: A - gene expression levels measured 1 day after application, B - gene expression levels measured 1 week after application. Calculations were normalized to the related gene expression levels measured in untreated cells; bars represent medians of the measurements, and different shapes correspond to different samples. Star (*) denotes statistically significant (p <0.05) differences, and two stars (**) denotes statistically significant (p <0.01) differences between the groups, evaluated via Conover-Iman test.

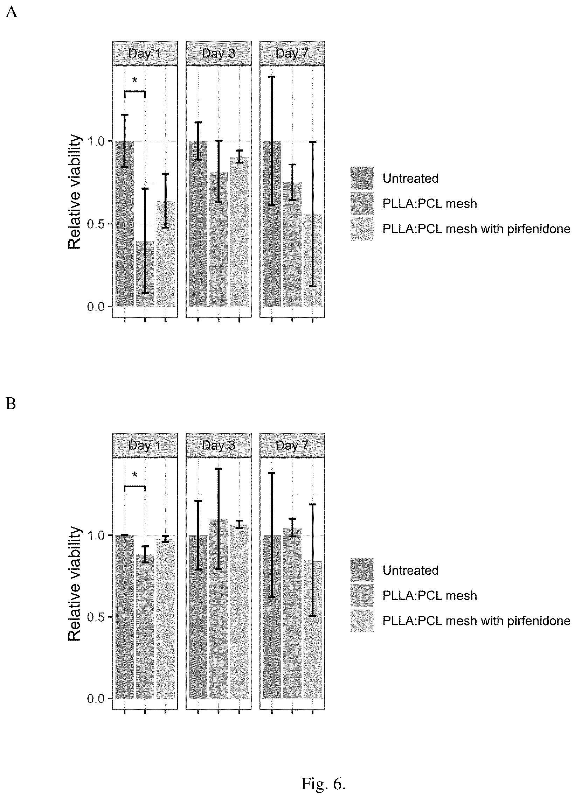

Fig. 6. shows the MTT measurement results, representing the relative viability of myofibroblast cells treated with directly applied pirfenidone and pirfenidone presented in the polymer nanofiber mesh. A - results of experiments with HMF cells, B - results of experiments with WPMY cells. Means (columns) and SD (error bars) displayed. Star (*) denotes statistically significant (p <0.05) differences between the groups, evaluated via t test.

### DETAILED DESCRIPTION OF THE INVENTION

The present invention relates to advantageous electrospun fiber compositions comprising one or more polymer and one or more therapeutically active molecules, wherein the one or more polymer is a copolymer of poly-lactic acid and poly-caprolactone in a molar ratio of 90:10. Accordingly, the present disclosure relates in particular to these electrospun fiber compositions, methods involving their use, as well as method of preparation of such fiber compositions.

### Drug-loaded electrospun fiber compositions

As used herein, "electrospinning" is a process in which fibers are formed from a solution or melt by streaming an electrically charged solution or melt through a hole across a potential gradient. The conventional electrospinning device has been widely modified to generate nanofiber filaments with the fiber diameters less than 1000 nm (Wu et al., 2022). "Fibers" are polymeric structures fabricated preferably using a biodegradable polymer for various medical applications such as surgical sutures, tissue regeneration and drug delivery. Accordingly, as used herein, "electrospun" material is any molecule or substance that forms a structure or group of structures (such as fibers, webs, or droplet), as a result of the electrospinning process. Electrospinning can be uniaxial or coaxial depending on the number of different materials and dispensers used for the process. The materials may be natural, synthetic, or a combination of such. The electrospun material may be in a form of a mesh of fibers. The mesh formation capabilities of the electrospinning method allow construction of structures each with an appropriate area and thickness used for specific cases.

Various polymers can be used in electrospun fiber compositions. According to the present disclosure, in particular synthetic biodegradable polymers such as polylactic acid (PLA) and poly-epsilon-caprolactone (PCL) are useful.

PLA is a polyester made from renewable biomass, typically from fermented plant starch like corn, cassava, sugarcane or sugar beet pulp. PLA is divided into three main sub-families, namely PDLLA (poly (DL-lactic acid)), PLLA (poly(L-lactic acid)) and PDLA (poly(D-lactic acid)). These three sub-groups of PLA have got the same chemical makeup but differ in their 3-dimensional molecular structure. PLLA (linear formula [-OCH(CH3)CO-]n, m.w. 325,000-460,000) is a biodegradable polymer for medical device and pharmaceutical applications. PLLA-based scaffolds can be exposed to surface modification or combined with other biomaterials, such as natural or synthetic polymers and bioceramics. Due to its relatively low biodegradation rate, PLLA may be a good candidate material for a prolonged drug delivery system. The chemical structure of PLLA (http://www.polysciences.com/india/polyl-lactic-acid-mw-325000-460000):

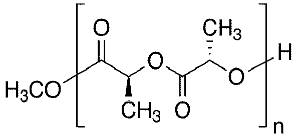

PCL (linear formula: (C6H10O2)n; average m.w. -14,000) is a semi-crystalline, aliphatic, biodegradable polyester with a low melting point of around 60 °C and a glass transition temperature of about -60 °C. PCL is prepared by ring-opening polymerization of ε-caprolactone using a catalyst such as stannous octanoate. The chemical structure of PCL (McKeen , 2021):

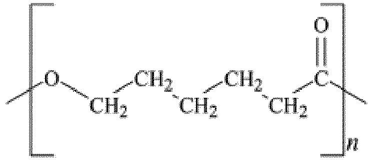

Under physiological conditions such as in the human body, PCL is degraded by hydrolysis of its ester linkages. The properties of PCL make it suitable for use as an implantable biomaterial.

The biodegradable polymers that are used to produce an electrospun fiber composition according to the present invention are copolymers poly-lactic acid (PLA) and poly-caprolactone (PCL). Preferably, a PLLA-PCL copolymer may be used. Its linear formula: (C4H6O3)m(C6H8O4)n. Like its components it is biodegradable. The chemical structure of PCL (https://www.sigmaaldrich.com/LT/en/product/aldrich/906840):

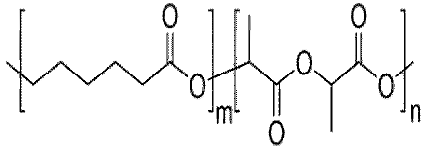

Accordingly, the electrospun fiber composition according to the present disclosure comprises a copolymer of poly-lactic acid (PLA) and poly-caprolactone (PCL). In particular, an electrospun fiber composition comprising one or more polymers and one or more therapeutically active molecules, wherein the one or more polymer is a copolymer of poly-lactic acid and poly-caprolactone in a molar ratio of 90:10, is provided. Such fiber composition comprising PLA:PCL in a molar ratio of 90:10 provides a beneficial therapeutically active molecule release pattern, in particular, provides prolonged supply of the therapeutically active molecules to the tissue or cells. In addition to the PLA-PCL copolymer, the fiber composition may further comprise other polymers, such as polyvinylalcohol (PVA), polylactic-co-glycolic acid (PLGA) or other similar molecules.

As used herein, the term "therapeutically active molecules", "drug" or "drug agents" refers to molecules, encompassing small molecule drugs, derivatives, analogs, and salts thereof, further including peptides, proteins, nucleic acids, carbohydrates, and other biologicals. In particular, a drug may be selected in the group comprising anti-inflammatory, anti-fibrotic, anti-microbial agent. The anti-inflammatory agents may be, for example, lidocaine or diclofenac. The anti-fibrotic agents may be, for example, nintedanib or pirfenidone. The anti-microbial agents may be, for example, gentamycin, tetracycline, ciprofloxacin.

In some embodiments, the electrospun fiber composition comprises a copolymer of poly-lactic acid and poly-caprolactone in a molar ratio of 90:10 and one or more therapeutically active molecules selected from anti-inflammatory, anti-fibrotic and anti-microbial agents. In some embodiments, the electrospun fiber composition comprises an anti-fibrotic agent. In preferred embodiments, the anti-fibrotic agent is pirfenidone (PFD).

In some embodiments, electrospun fiber composition of the present invention comprises one or more therapeutically active molecules at a concentration of 0.3-3% (w/w). In some embodiments, the one or more therapeutically active molecules are present at a concentration selected from 0.3%, 0.4%, 0.5%, 0.6%, 0.7%, 0.8%, 0.9%, 1%, 1.1%, 1.2%, 1.3%, 1.4%, 1.5%, 1.6%, 1.7%, 1.8%, 1.9%, 2%, 2.1%, 2.2%, 2.3%, 2.4%, 2.5%, 2.6%, 2.7%, 2.8%, 2.9%, and 3%, (w/w). In some embodiments, each of the therapeutically active molecules is present at a concentration of 0.3-3% (w/w). In other embodiments, the overall concentration of the therapeutically active molecules is 0.3-3% (w/w). In some embodiments, the therapeutically active molecule is present at a concentration of 1% (w/w). In some embodiments, the therapeutically active molecule is pirfenidone, that is present at a concentration of 1% (w/w).

The electrospun fiber composition as provided in the present disclosure may be in the form of a fiber delivery platform, a fiber dressing, or a fiber scaffold. As used herein, "fiber delivery platform" is a fiber composition suitable to be used internally for delivery of molecules to the surrounding tissues. "Fiber dressing", as used herein, is a fiber composition suitable to be used externally for delivery of molecules to cutaneous wounds, tumours or other types of skin lesions or damaged tissues. "Fiber scaffold", as used herein, is a fiber composition for delivery of molecules to surrounding tissues with the additional function of structural support for additional tissue engineering materials, such as polymers, decellularized tissues, bioprinted extracellular matrices or other materials attached to the composition. The fiber composition of the present invention may be provided in other forms, such as, e.g., medical dressing or a wound dressing patch, that are desired in application where drug delivery and release carriers are desired. A netted or mesh structure is preferred, such that the fiber composition may be conveniently implanted as an inlay, for example, inside of or onto the tissue.

### Methods of use

Provided herein are also the methods of use of the fiber composition according to the present invention.

In some embodiments, a pharmaceutical composition comprising the fiber composition according to the present invention and a pharmaceutically acceptable carrier is provided.

In other embodiments, the present disclosure provides a fiber composition according to the present invention for use in a method of treatment of wounds and/or fibrotic tissue. The fiber composition may be sutured onto, glued into or otherwise immobilized or fixed to the tissue. In some embodiments, a mammal tissue is treated, for example, a tissue of a mouse, a rat or a rabbit. In some embodiments, a fiber composition according to the present invention is for use in a method of treatment of wounds and/or fibrotic tissue, wherein the tissue is a human tissue.

Further uses of the fiber composition of the present invention comprise the use of the composition for delivery of one or more therapeutically active molecules to the cells. The cells may be selected from primary or line stem cells, cancer cells or proliferating tissue cells suitable for work in a cell culture laboratory. In some embodiments, in the methods of the present invention the cell are eukaryote cells. In some embodiments, the cells are non-human animal cells. In other embodiments, the cells are human cells.

In some embodiments, the method of treatment where the fiber composition may be used is a method for treatment of fibrotic tissue of urethral strictures or hypospadias. In particular, the fiber composition according to the present invention, comprising anti-fibrotic agent, is useful in such method. More particularly, pirfenidone (PFD) is used in such fiber composition.

The main tissue effector in fibrosis is the myofibroblast cell. During fibrosis myofibroblasts proliferate intensively and produce large quantities of extracellular matrix that often results in destruction of normal architecture of tissue and causes significant organ dysfunction. TGF- β1 (transforming growth factor- β1) is known to play a central role in fibrosis pathogenesis. It is a crucial regulator of fibroblast phenotype and function. Upon TGF-β stimulation, fibroblasts are activated and undergo phenotypic transition into myofibroblasts being the key effector cells in fibrotic states (Biernacka et al., 2011). Due to its size and hydrophobic nature PFD is able to diffuse freely across cell membranes without using a receptor. PFD acts by suppressing the induction of fibrogenic mediators and growth factors through a variety of mechanisms (Ruwanpura et al., 2020). It impacts TGF- β1 and downstream pathways by reducing TGF- β1 protein production (Ma et al., 2018) and slightly attenuated TGF-β1 induced expression of fibronectin and α-smooth muscle actin in fibroblast cultures, without impairing cell viability (Stahnke et al., 2018). Moreover, PFD significantly suppresses TGF-β1-induced ECM synthesis (Cui et al., 2020).

In some embodiments, the fiber composition is used in a method for treatment of fibrotic tissue of urethral strictures or hypospadias, wherein the fibrotic tissue is of a mouse, a rat or a rabbit. In other embodiments, the fibrotic tissue is a human tissue.

### Method for the preparation of the fiber composition

Further, a method for the preparation of the fiber composition according to the present invention is provided, the method comprising:
1) preparing a solution of a copolymer poly-lactic acid and poly-caprolactone and one or more therapeutically active molecules, and
2) subjecting the solution to an electrospinning step,
wherein the solution of step 1) is prepared in dichlormethane (DCM).

The method is useful in that only a minimal number of components is used - copolymer, therapeutically active molecule and solvent DCM. Advantageously, no additional steps apart from the preparation of a solution as in step 1) are needed before the electrospinning step can be done.

In some embodiments, in the hereby disclosed method the copolymer is present at a concentration of 5-20% (w/v), and the one or more therapeutically active molecule is present at a concentration of 0.03-0.3% (w/v).

### EXAMPLES

### Example 1. Preparation of PLA-PCL fiber composition

Various blends of PLLA and PCL polymers, as well as pure PCL can be used to prepare the substrate for electrospinning the mesh for PFD delivery. In this case, polymers as well as the carried PFD molecule are dissolved and mixed in dichlormethane (DCM). The procedure for preparing the mixture for electrospinning was as follows:
1. 900 mg of polymer or polymer blend was weighed into a glass bottle with stopper.
2. 9 mg of PFD was weighed and added to the bottle.
3. 9.1 ml of DCM was poured on the polymer and PFD, making the final substrate concentration 9% w/v polymer and 0.09% w/v PFD. The bottle was closed firmly to prevent evaporation.
4. The mixture was left to dissolve for 4 h at room temperature, with vigorous mixing with a glass rod every 1 h. The final appearance of the substrate should be a clear, viscous fluid with no particulate contamination.

The prepared mixture was used for electrospinning. RegenHu bioprinter device was used for electrospinning, the parameters for the device were as follows:
1. Plunger speed: 0.08 µl/s
2. Dispenser movement speed: 10 mm/s
3. Dispenser height: 5 cm
4. Platform voltage: 25 kV

As a result, a mesh with fibers in the micron range was produced (Fig 1). Depending on the area covered by the dispenser, the dimensions of the mesh can be controlled. The thickness of the mesh can be controlled by performing the electrospinning for a longer time. Advantageously, DCM was sufficient as a single solvent for dissolving the components when preparing the mixture for electrospinning, and the electrospinning was efficient.

Mesh formation by electrospinning capabilities allow construction of personalized structures each with an appropriate area and thickness. This is useful for specific cases, for example, in surgery aimed at restoration of damaged urethral tissue both externally and internally.

### Example 2. Pirfenidone release study

The pirfenidone (PFD) release study was conducted by placing the polymer samples in 40% ethanol solution in PBS. 100 mg of the samples (containing 1 mg of PFD) were submerged in 200 µl of this buffer and incubated at 37°C. Absorbance of PFD in the buffer collected from the samples at the specified time points was measured using a spectrophotometric plate reader. Concentration was calculated using a PFD standard curve prepared in the test buffer. The buffer was replenished to maintain equal volume across samples and time points.

As demonstrated in Fig. 2, the release profile of PFD depends on the polymeric composition of the meshes (absorbance of PFD was measured by spectrophotometry, the concentration was calculated according to standard curve measurements). The polymer with composition of PLLA:PCL (90:10) allows maintenance of a therapeutic concentrations of PFD, >0.1 mg/ml, in cell culture dishes for up to 9 weeks following initial application, which is substantially better than the other polymer compositions tested (e.g., 60:40).

The PFD release profile by the PLLA:PCL 90:10 mesh structure was compared to a cast film of the same polymer-PFD suspension. The film was manufactured by pouring 1 ml of a suspension comprising PLLA:PCL (90:10) copolymer and PFD and allowing the DCM to evaporate, producing a 100 mg of smooth film. Both the mesh and the film samples were submerged in the ethanol-PBS buffer and the cumulative release of PFD was evaluated by measuring the concentration of PFD at the specified time points and calculating the ratio to the cumulative mass of PFD used when manufacturing the samples. The resulting PFD release profile of the mesh was significantly different from the data for the cast PLLA:PCL (90:10) copolymer film (Fig. 3). The mesh released around 50% of total stored PFD at the 3 day mark and had mostly depleted the drug within 3 weeks, whereas the film released 50% of the PFD at the 5 day mark and had mostly depleted the drug at the last time point measured (35 days). Therefore, the pirfenidone-loaded copolymer mesh was clearly more efficient in releasing PFD within the crucial first three weeks of application.

In long-term and clinically relevant studies, it is important that the stimulation by PFD is continued for weeks or longer, therefore, the use of PFD-carrying mesh as disclosed herein, is beneficial, as opposed to the direct application of PFD that resulted in a quick loss of the molecules as they degrade or break apart in the medium.

The slow release of PFD from a polymeric mesh in comparison to its direct application has a distinct and beneficial effect both *in vivo* treatment and *in vitro* research. The mesh structure of the polymeric carrier ensures a stable diffusion of PFD molecules into the surrounding environment. Large-surface fibers facilitate drug diffusion, while micro-scale fibers in the mesh structure facilitate passage of nutrients and cells in the tissue.

### Example 3. Effect of pirfenidone on the gene expression in the cells

In these studies, primary human myofibroblasts (HMF) were grown in FBS-supplemented DMEM culture media. The cells were grown in 12-well culture plates, with 1000 µl of the culture medium used. PFD was applied directly by adding 500 µg of PFD to the culture medium or presented within the mesh by submerging a 50 mg (with 500 µg PFD loaded) sample in the medium. Addition of PFD to the media stimulated an anti-fibrotic response in the cells.

The mesh manufactured using a copolymer with a mass ratio of PLLA:PCL = 90: 10, laden with PFD demonstrated the reduction of fibrosis-related gene expression markers in primary human myofibroblasts (HMF) (Fig. 4). In contrast to directly applied PFD, the application of the PFD-laden mesh reduces the expression of the pro-fibrotic gene TIMP1, as well as the expression of other pro-fibrotic genes - CTGF and PAI1 - is reduced (Fig. 4A). Compared to direct application of PFD, this anti-fibrotic effect is stronger when using the PFD-loaded mesh after 1 week of application (Fig. 4B), as witnessed in the sustained decrease of the pro-fibrotic PAIl gene expression. The results observed *in vitro* suggest a similar *in vivo* effect inside the body, thus the application of this mesh could lead to the suppression of scar formation, as it would attenuate the expression of pro-fibrotic genes.

The mesh can be used to investigate the effects of PFD in cancer cells, for example, prostate cancer, and treatment research can be carried out *in vitro* using cancer cell lines, such as WPMY-1. Prostate cancer stromal myofibroblasts (WPMY-1) were grown in FBS-supplemented DMEM culture media. The cells were grown in 12-well culture plates, with 1000 µl of the culture medium used. PFD was applied directly by adding 500 µg of PFD to the culture medium or presented within the mesh by submerging a 50 mg (with 500 µg PFD loaded) sample in the medium. As demonstrated in Fig. 5, the direct application of PFD has a different effect on the cells compared to its application via polymer mesh. Initially both treatments increase the expression of MMP1 gene (Fig. 5A). One week after treatment, regardless of whether free or mesh-carried PFD was used, the increase of TGFB 1 gene expression was observed (Fig. 5B). However, the directly applied PFD also elicits an increase in CTGF and VEGFA gene expression, while no such effect was observed when the PFD-laden mesh was applied. These proteins are linked to the initiation of fibrotic processes, and the increase in the expression of their genes should be avoided. Thus, it is shown that PFD-laden mesh can be used to stimulate a sustained-release effect of the drug which is distinct from the one-time direct application.

### Example 4. Effect of pirfenidone on the proliferation of myofibroblasts

In a manner similar to described in Example 3, HMF and WPMY-1 cells were seeded at a low density to culture plates and PFD was applied directly to the medium or via mesh. In these experiments, the mesh without loaded PFD was also tested (Fig. 6). The proliferation rate of the cells was measured by MTT assay. Suppression of cell proliferation (HMF, Fig. 6A; WPMY-1, Fig. 6B) was observed when they were treated with the non-PFD-laden mesh for 1 day, however, this suppression was no longer present after 3 or 7 days. Thus, it can be concluded that the meshes are not cytotoxic.

### Example 5. In vivo results

PFD loaded PLLA:PCL 90:10 meshes were tested in a rabbit experimental model *in vivo.* The mesh was implanted into the mid-urethral part of the rabbit. Under general anesthesia and sterile conditions midline incision was performed at ventral wall of penile urethra. 20x10 mm urethral defect was created and it was covered using a tissue graft with the PFD loaded mesh. A 3-month post-operative follow-up was performed, with monitoring, urine and blood samples, urethrograms. Monitoring included assessment of rabbit weight, temperature, secretions, behavior and wound healing. The experiment was concluded with a final histological examination. The analysis showed that using the PFD loaded mesh resulted in less fibrosis and less luminal inflammation of the urethra. Better long-term postoperative results, better welfare of animals, less expressed laboratory and histological signs of inflammation, leukocytosis and tissue infiltration were confirmed. In summary, using the PFD loaded provides promising advantage in urethral reconstructive surgery. The results provided herein show that the mesh serves as a stricture-repairing structure and simultaneously as a drug carrier. The mesh fibers made of the polymeric drug-carrier ensures a stable diffusion of pirfenidone molecules into the surrounding environment.

### References

Biernacka A., Dobaczewski M., Frangogiannis N.G. TGF-β signaling in fibrosis. Growth Factors. 2011; 29(5): 196-202. doi:10.3109/08977194.2011.595714
Cui Y., Zhang M., Leng Ch., Blokzij T., Jansen B.H., Dijkstra G., Faber K.N. Pirfenidone inhibits cell proliferation and collagen I production of primary human intestinal fibroblasts.
Cells. 2020, 9, 775. doi:10.3390/cells9030775
El-Kassaby A.W., Retik A.B., Yoo J.J., Atala A. Urethral stricture repair with an off-the-shelf collagen matrix. J. Urol. 2003; 169: 170-173. DOI: 10.1016/S0022-5347(05)64060-8
Elliott S.P., Virasoro R., Estrella R., et al. The Optilume Drug Coated Balloon for Recurrent Anterior Urethral Strictures: ROBUST I Clinical Study 3-year follow up, N = 43. J. Urol. 2021, 206(3S): e971.
Klekiel T., Mackiewicz A., Kaczmarek-Pawelskaa A., Skonieczna J., Kurowiak J., Piasecki T., Noszczyk-Nowakb A., Bedzinski R. Novel design of sodium alginate based absorbable stent for the use in urethral stricture disease. J. Mater. Res. Technol. 2020, 9, 4, 9004-9015.
DOI:10.1016/j.jmrt.2020.06.047
Kuo H.-Ch. Clinical application of botulinum neurotoxin in lower-urinary-tract diseases and dysfunctions: where are we now and what more can we do? Toxins (Basel). 2022, 14(7): 498. doi: 10.3390/toxins 14070498
Ma Y., Jian Z.Y., Hu O., Luo Zh., Jin T. Oral Mucosa vs. Penile skin flap in substitution urethroplasty for anterior urethral strictures: a systematic review and meta-analysis. Front. Surg. 2021; 8: 803750. doi: 803750. 10.3389/fsurg.2021.803750
Ma Z, Zhao C, Chen Q, Yu C, Zhang H, Zhang Z, et al. Antifibrotic effects of a novel pirfenidone derivative in vitro and in vivo. Pulm. Pharmacol. Ther. 2018, 53: 100-106. DOI: 10.1016/j.pupt.2018.10.006
McKeen L., The effect of heat aging on the properties of sustainable polymers, in The effect of long term thermal exposure on plastics and elastomers. 2021. doi.org/10.1016/B978-1-4557-2598-4.00012-5
Mundy A.R. Management of urethral strictures. Postgrad. Med. J. 2006, 82:489-493. doi: 10.1136/pgmj.2005.042945
Proesmans V.L.J, Drent M., Elfferich M.D.P., Wijnen A.H.M., Jessurun N.T., Bast A. Self-reported gastrointestinal side effects of antifibrotic drugs in Dutch idiopathic pulmonary fibrosis patients. Lung. 2019; 197(5): 551-558. doi: 10.1007/s00408-019-00260-1
Rashidbenam Z., Jasman M.H., Hafez P., Tan G.H., Goh E.H., Fam X.I., Ho Ch.Ch.K., Zainuddin Z.M., Rajan R., Nor F.M., Shuhaili M.A., Kosai N.R., Imran F.H., Ng M.H.N. Overview of urethral reconstruction by tissue engineering: current strategies, clinical status and future direction. Tissue Eng. Regen. Med. 2019, 16(4): 365-384. doi.org/10.1007/s13770-019-00193-z
Ruwanpura S.M., Thomas B.J., Bardin P.G. Pirfenidone: molecular mechanisms and potential clinical applications in lung disease. Am. J. Respir. Cell Mol. Biol. 2020, 62(4): 413-422. doi: 10.1165/rcmb.2019-0328TR
Sack B.S., Mauney J.R., Estrada CR Jr. Silk fibroin scaffolds for urologic tissue engineering. Curr. Urol. Rep. 2016, 17(2): 16. doi: 10.1007/sll934-015-0567-x
Simsek A., Aldamanhori R., Chapple Ch.R., MacNeil sh. Overcoming scarring in the urethra: Challenges for tissue engineering. AJUR. 2018, 5, 69e77. doi.org/10.1016/j.ajur.2018.02.002. Simonato A., Gregor A., Ambruosi C., Venzano F., Varca V., Romagnoli A., Carmignani G. Lingual mucosal graft urethroplasty for anterior urethral reconstruction. Eur. Urol. 2008, 54(1): 79-85. doi: 10.1016/j.eururo.2008.01.023
Srivastava C.A., Dutta A., Jain D.K. Initial experience with lingual mucosal graft urethroplasty for anterior urethral strictures. MJAFI. 2013, 69 (1), 16-20. doi.org/10.1016/j.mjafi.2012.05.006 Stahnke Th., Kowtharapu B.S., Stachs O., Schmitz K.-P., Wurm J., Wree A., Guthoff R.F., Hovakimyan M. Suppression of TGF-β pathway by pirfenidone decreases extracellular matrix deposition in ocular fibroblasts in vitro. PLoS ONE 12(2): e0172592.
doi:10.1371/journal.pone.0172592
Virasoro R., DeLong J.M., Estrella R.E., Pichardo M., Rodriguez Lay R., Espino G., Elliott S.P. A Drug-coated balloon treatment for urethral stricture disease: three-year results from the ROBUST I study. Res. Rep. Urol. 2022, 14, 177-183. doi.org/10.2147/RRU.S359872
Wu S, Dong T, Li Y, Sun M, Qi Y, Liu J, Kuss MA, Chen S, Duan B. State-of-the-art review of advanced electrospun nanofiber yarn-based textiles for biomedical applications. Appl. Mater. Today. 2022, 27: 101473. doi: 10.1016/j.apmt.2022.101473)

## Claims

1. An electrospun fiber composition comprising one or more polymers and one or more therapeutically active molecules,
wherein the one or more polymer is a copolymer of poly-lactic acid and poly-caprolactone in a molar ratio of 90:10.

2. The fiber composition according to claim 1, wherein the one or more therapeutically active molecules are selected from anti-inflammatory, anti-fibrotic and anti-microbial agents.

3. The fiber composition according to claim 2, wherein the anti-fibrotic agent is selected from nintedanib and pirfenidone.

4. The fiber composition according to claim 3, wherein the anti-fibrotic agent is pirfenidone.

5. The fiber composition according to any one of the claims 1 to 4, wherein the one or more therapeutically active molecule is present at a concentration of 0.3-3% (w/w).

6. The fiber composition according to claim 5, wherein the one or more therapeutically active molecule is present at a concentration of 1% (w/w).

7. The fiber composition according to any one of the claims 1 to 6, wherein poly-lactic acid is selected from poly-L-lactic acid and poly-D-lactic acid.

8. The fiber composition according to claim 7, wherein poly-lactic acid is poly-L-lactic acid.

9. The fiber composition according to any one of the preceding claims, wherein the composition is in the form of a fiber delivery platform, a fiber dressing, or a fiber scaffold.

10. Pharmaceutical composition comprising the fiber composition according to any preceding claims and a pharmaceutically acceptable carrier.

11. The fiber composition according to any one of the preceding claims for use in a method of treatment of wounds and/or fibrotic tissue.

12. The fiber composition, wherein the method is for treatment of fibrotic tissue of urethral strictures or hypospadias.

13. Use of the composition according to any one of the preceding claims for delivery of one or more therapeutically active molecules to the cells.

14. A method for the preparation of the fiber composition according to any one of claims 1 to 12, comprising:
1) preparing a solution of a copolymer poly-lactic acid and poly-caprolactone and one or more therapeutically active molecules, and
2) subjecting the solution to an electrospinning step,
wherein the solution of step 1) is prepared in dichlormethane.

15. The method according to claim 14, wherein the copolymer is present at a concentration of 5-20% (w/v), and the one or more therapeutically active molecule is present at a concentration of 0.03-0.3% (w/v).
